(19) Europäisches Patentamt
European Patent Office
Office européen des brevets

(11) Publication number: **0 556 699 A1**

(12) # EUROPEAN PATENT APPLICATION

(21) Application number: **93102016.8**

(22) Date of filing: **09.02.93**

(51) Int. Cl.[5]: **C12N 15/52**, C12P 17/06, C12N 15/80, C12P 7/42, C12N 1/15, //(C12N1/15, C12R1:66)

(30) Priority: **10.02.92 US 833496**
**27.02.92 CA 2062023**

(43) Date of publication of application:
**25.08.93 Bulletin 93/34**

(84) Designated Contracting States:
**AT BE CH DE DK ES FR GB GR IE IT LI LU MC NL PT SE**

(71) Applicant: **NOVOPHARM LTD.**
**30, Nably Court**
**Scarborough, Ontario M1K 2B4(CA)**

(72) Inventor: **Dahiya, Jagroop S.**
**33-2088 Pembina Highway, Winnipeg**
**Manitoba R3T 2G8(CA)**

(74) Representative: **Müller-Boré & Partner Patentanwälte**
**Isartorplatz 6 Postfach 26 02 47**
**D-80059 München (DE)**

(54) **Novel fungal strains and use thereof in antibiotic production.**

(57) Lovastatin is produced by a process of fermentation using a fungal transformant produced by introducing into a non-lovastatin expressing Aspergillus strain such as a strain of Aspergillus oryzae the DNA of a lovastatin-expressing strain of Aspergillus terreus.

EP 0 556 699 A1

This invention relates to novel, genetically engineered fungal strains, and use thereof in preparation of antibiotics. More specifically, it relates to novel, genetically engineered strains of Aspergillus and their use in preparation of the drug lovastatin and analogs thereof.

Lovastatin is, chemically [1S-(1α(R*),3α,7β,8β (2S*,4S*), 8αβ]]-2-methylbutanoic acid 1,2,3,7,8,8a-hexahydro-3,7-dimethyl-8-[2-(tetrahydro-4-hydroxy-6-oxo-2H-pyran-2-yl)-ethyl]-1-naphthalenyl ester, and has the chemical formula:

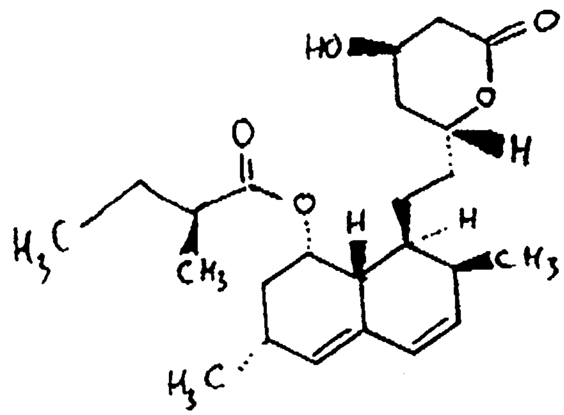

It is useful as an antihypercholesterolemic, being a potent inhibitor of HMG-CoA reductase, the rate controlling enzyme in cholesterol biosynthesis. It is a fungal metabolite produced by fermentation processes using selected fungal strains.

Antibiotics such as lovastatin are metabolites which require sets of several enzymes for their synthesis. To permit their production by molecular cloning of antibiotic-producing microorganisms requires the isolation, analysis and, perhaps modification of the corresponding genes for the several enzymes. Attempts to isolate such genes from such fungal species have so far yielded clones carrying either individual genes of the set, or only incomplete gene sets - see Malpartida and Hopwood, "Molecular Cloning of the Whole Biosynthetic Pathway of a Streptomyces Antibiotic and its Expression in a Heterologous Host", Nature (1984), 309 pp 462-464.

Canadian Patent 1,129,794 Endo, describes the preparation of lovastatin by fermentation using a strain of the fungal species Monascus ruber.

Canadian Patent 1,161,380 Monaghan et al., describes the preparation of lovastatin by fermentation using strains of the fungal species Aspergillus terreus.

In both of these prior art processes, lovastatin is produced along with other, very similar chemical compounds, in substantial quantities, from which it must be separated. In its production using Monascus ruber, lovastatin co-occurs with monacolin J. In its production using Aspergillus terreus, it co-occurs with dihydrolovastatin and with the hydroxy acid. Whilst the hydroxy acid can be readily lactonized to lovastatin, the dihydrolovastatin must be separated from it.

It is an object of the present invention to provide novel, genetically engineered mutant fungal strains capable of use in fermentation processes to make lovastatin.

It is a further object of the invention to provide novel processes for making lovastatin by fermentation using such strains.

According to one aspect of the present invention, novel mutant fungal strains of appropriate species of the genus Aspergillus are provided, which are capable of expressing and secreting lovastatin. These strains contain the genes of the lovastatin-producing enzyme set, in functioning relationship, derived from the DNA of lovastatin-producing Aspergillus terreus strains. They are produced by a process of protoplast transformation of the DNA from the lovastatin-producing Aspergillus terreus strain, also containing an appropriate selectable marker, with another, non-lovastatin producing Aspergillus species, selected from A. oryzae, A. fumigatus, A. niger, A. nidulans and A. flayus, selection of the transformants so formed on the basis of the selectable marker, identification and isolation of the lovastatin-producing transformants and sub-cloning thereof.

According to another aspect of the invention, there is provided a process of preparing lovastatin which comprises fermenting a nutrient medium with a transformant Aspergillus microorganism containing genes derived from Aspergillus terreus and coding for the set of lovastatin-producing enzymes, and recovering the

lovastatin so formed.

The process of the present invention produces lovastatin with significantly smaller amounts of contaminating dihydrolovastatin and hydroxy acid derivatives, as compared with processes using Aspergillus terreus.

In the process of making the transformants of the present invention, standard techniques of preparing protoplasts from the selected, non-lovastatin producing Aspergillus strain, and standard techniques of extracting the DNA from the lovastatin-producing Aspergillus terreus strain can he employed. These techniques are well known to those of skill in the art and do not need detailed discussion herein. Similarly, the techniques and procedures of protoplast transformation useful herein are known and standard.

The preferred choice of non-lovastatin producing Aspergillus strain is a strain of Aspergillus oryzae, but this in not critical to success in practising the invention. Other species of Aspergillus namely A. niger, A. nidulans, A. fumigatus and A. flavus, can be used. A. oryzae is chosen as the preferred species on account of its inertness, which renders it easy and safe to handle in the laboratory and in commercial scale fermentations.

In order to select transformant microorganisms from non-transformants after the protoplast transformation process, the DNA from the A. terreus should contain a selectable marker. There is a wide choice of selectable markers available to and selectable by the skilled worker, and the precise choice is not critical to success in working the invention. Markers of antibiotic resistance such as ampicillin resistance, rifampicin resistance, streptomycin resistance etc. can be used, and the resulting mixture of transformants and non-transformants can be cultured in a medium containing the appropriate antibiotic, so that only the transformants, which contain the selectable marker, will survive for isolation.

Particularly preferred as a selectable marker, on the basis of convenience, is cycloheximide resistance. Cycloheximide is an inhibitor of protein synthesis, so that the presence of a cycloheximide-resistant strain or transformant in a culture broth is readily detected.

After selection of the transformants on the basis of the selectable marker, they are screened for those which will express and secrete lovastatin. Only a relatively small number of the total transformants produced in the protoplast transformation process have this capability. They are recognized by separate culturing in standard culture broth, and analysis of the resulting medium, e.g. by HPLC, for the presence of lovastatin. Those testing positive for the presence of lovastatin are sub-cultured and grown, to yield colonies of novel, lovastatin-producing transformant fungal microorganisms of the Aspergillus genus and preferably of the Aspergillus oryzae species.

The invention is further described for illustrative purposes in the following experimental accounts.

In the accompanying drawings:

FIGURE 1 is a graphical presentation of the HPLC analysis of the crude fungal extracts produced according to the Example 1 experiments described below;

FIGURE 2 is the $^{1}$H-NMR spectrum of the lovastatin compound obtained and purified from the hybrid strain;

FIGURE 3 is the mass spectrum of the same compound;

FIGURE 4 is a depiction of the morphological characteristics of novel transformant AoAt/NBJ-V.

## EXAMPLE 1

### MATERIALS AND METHODS

FUNGAL CULTURE - fungal isolates Aspergillus terreus and A. oryzae used in the present study were isolated from orchid soil samples collected from Agriculture Canada, Research Station, Beaverlodge, Alberta, Canada.

### SELECTION OF CYCLOHEXIMIDE RESISTANT MUTANTS

Having grown both Aspergilli isolates on Czapek's dox media containing cycloheximide (conc. 0.1-5mM) for 6 days at 28 ± 2°C, Spores ($10^{8}$) of each isolate A. terreus and A. oryzae were dispensed on 10 mM cycloheximide Czapek's dox broth (50 ml) and incubated for 30 min., centrifuged at 10,000 g for 10 min., and then re-suspended in sterile distilied water. They were washed three times by agitation and resedimented by centrifugation. The spores were then suspended in sterile 1%(v/V) Triton X-100 solution and their numbers determined in an aliquot on a haemocytometer. An appropriate dilution was dispensed on the 10mM cycloheximide selection medium and resistant mutants were isolated after 10 days of incubation at 28 ± 2°C. Isolates were examined for lovastatin production. A. cycloheximide resistant lovastatin producing

isolate of A. terreus was selected for further transformation study.

## PROTOPLAST AND DNA PREPARATION

Aspergillus oryzae and the cycloheximide-resistant lovastatin-producing A. terreus (herein designated JAG-4703) isolate were cultured separately in 50 ml of Czapek's dox broth. The cultures were incubated for 58 hours at 28 ± 2°C on a rotary shaker (New Brunswick Scientific Inc.) at 200 rpm, then harvested and washed with sterile distilled water by repeated centrifugation.

Protoplasts from cultures of both species were obtained by using Novozym 234 (Novo-Nordisk, Novo Alle, DK 2880, Bagsvaerd, Denmark) to remove the cell walls during 10-12 hour digestion, generally following the method described by Dickinson & Isenberg, J. Gen. Microbiol. 128, p. 651-654 (1982). Aliquots of protoplasts from each species regenerated viable, single cell walled spores at 28°C after 24 hours in regenerating medium(R) containing 0.1g agar (Difco) 5g sorbose and 0.35g EDTA in 50 ml distilled water. On germination these spores developed all the cultural characteristics of their parents.

The total DNA was isolated from Aspergillus terreus protoplasts according to the procedure described by Schlief & Wensink, "Practical Methods in Molecular Biology", 33, 21-29 (1981). The protoplasts were suspended in a solution consisting of 10 mg/ml SDS,0.1M NaCL and 0.1M tris-HCL, pH 9.0. An equal volume of phenol saturated with the tris-HCL buffer was added. The mixture was centrifuged at 12,000 g for 10 min. in an Eppendorf Centrifuge tube (Brinkmann Instruments, Canada Ltd., Rexdale, Ontario). The upper phase, containing the DNA, was removed, mixed with 95% ethanol, stored at -20°C for 60 min., then centrifuged as before for 10 min. The pellet was re-suspended in buffer, pH 7.0, and incubated with RNase (Sigma, St. Louis, MO, U.S.A.) treated with phenol and the DNA precipitated from the aqueous layer. The isolated DNA was purified by adsorption and washing on DEAE-cellulose (DE-52, Whatman), presoaked in 10mM tris-HCL buffer, pH 7.5 and 0.3M NaCL and contained in a pasteur pipette. The DNA was eluted with 10mM tris-HCL buffer, pH 7.5 containing 1.5M NaCL. This solution was diluted to 0.2M NaCL and the DNA was precipitated with two volumes of ethanol. The purity of the DNA was estimated from the 200-320 nm spectra by determining the $A_{260}/A_{280}$ absorbance ratio. Only DNAs with ratios between 1.50 and 2.00 were used in the transformations. Six samples, each 0.1 mg of the isolated DNA were incubated in the regenerating medium to ensure the absence of viable protoplasts and none of them developed any colony.

## PROTOPLAST-DNA INCUBATION

Approximately $5.2 \times 10^4$ A. oryzae protoplasts, estimated by haemocytometer counts, were incubated with 10-100 ng of A. terreus DNA in 5 ml regenerating medium, the composition of which was as described above, and regenerated as described above. In order to study the possible chemical effects of DNA on lovastatin expression, 10-100 ug calf-thymus DNA (Sigma Chemical Co., St. Louis, MO., U.S.A.), DNA (Bethesda Research Laboratories, Gaitherberg, MD, U.S.A.) and homologous A. orzyae DNA were incubated with similar lots of A. oryzae protoplasts. When DNA was included in the incubations, protoplast regeneration was reduced to less than 10% and comparable numbers of such treated protoplasts ($10^5$) failed to yield any cycloheximide resistance.

## LOVASTATIN PRODUCTION AND ASSAY

The suspension of spores developed from A. oryzae protoplasts incubated with A. terreus DNA was inoculated, 1ml per petriplate, onto Czapek'dox agar medium containing 10mM cycloheximide. After incubation for 48 hours at 28 ± 2°C, cultures attained a diameter of 6-8 mm. Each developed separately from a single spore under these conditions. Those which grew on the cycloheximide medium were each inoculated separately onto Czapek'dox media broth (50ml in 500ml Erlenmeyer flask), incubated on rotary shaker (200 rpm) at 28 ± 2°C for 12 days. Each growth media were examined for lovastatin production by the procedure described below and HPLC analysis.

## EXTRACTION

Fermented broth (50ml) was acidified to pH 4.0 with 17N HCL and then fractionated against ethylacetate (100ml, 3x). Pooled ethylacetate fractions were dried in vacuo at 30°C, the residue was collected in acetonitrile (5ml) and then subjected to HPLC analysis.

HPLC ANALYSIS

The HPLC equipment (Beckman Model 420) was supplied by Beckman Instruments, Toronto, Canada and consisted of an Altex pump (Model 110 A) and injection valve. The LC-UV detector was set at 237 nm. A hypersil C-18 ODS silica column (25 x 0.46cm, i.d.) and a solvent system of acetonitrile and water (55:45,v/v) at flow rate of 1.0 ml/min. were used. Lovastatin produced was compared and quantitated from a standard lovastatin curve constructed.

EXAMPLE 1 - RESULTS

Seventy-nine cycloheximide-resistant isolates, including four lovastatin-producing isolates, were obtained from 5.2 x $10^5$ A. oryzae protoplasts incubated with the DNA from the cycloheximide-resistant lovastatin-producing mutant of A. terreus. The transformation experiments were performed six times.

The results are given in the following Table 1.

**TABLE 1** Cycloheximide-resistant and lovastatin-expressing *Aspergillus oryzae* protoplasts incubated with DNA from the cycloheximide-resistant lovastatin-producing mutant of *Aspergillus terreus*.

| Expt.# | Protoplasts.ml | Number of protoplasts Regenerated, After Protoplast Fusion, #/ml | % Regeneration | DNA (ng) | Number of Lovastatin-Producing Isolates |
|---|---|---|---|---|---|
| i) | 7.4 x $10^5$ | 4.1 x $10^6$ | 56 | 100 | 0 |
| ii) | 5.2 x $10^6$ | 3.1 x $10^5$ | 60 | 75 | 2 |
| iii) | 5.2 x $10^6$ | 3.7 x $10^6$ | 72 | 50 | 2 |
| iv) | 4.0 x $10^5$ | 2.7 x $10^5$ | 70 | 25 | 0 |
| v) | 2.6 x $10^6$ | 1.5 x $10^5$ | 65 | 10 | 0 |
| vi) | 1.6 x $10^5$ | 1.1 x $10^5$ | 70 | 0 | 0 |

One of the transformed cultures AO-II, retained its original transformed characteristics for six months. Fig. 1 illustrates the HPLC analysis of the extracts obtained from the recipient and donor cultures of A. oryzae, and those treated with A. terreus DNA. The production of lovastatin by transformed isolates is shown below in Table 2.

TABLE 2

| Lovastatin production by transformed isolates of Aspergillus oryzae in Czapek'dox broth (pH 6.8) | |
|---|---|
| Isolate # | Lovastatin Yield (mg/1) |
| A0-I | 15.36 ± 1.78 |
| AO-II | 26.89 ± 3.76 |
| AO-III | 13.46 ± 4.56 |
| AO-IV | 9.67 ± 0.75 |

Isolate no. AO-I was sub-cultured five times, to yield a transformant AoAt- NBJ/5 which has been deposited on February 25, 1992 as a viable, permanent culture thereof with American Type Culture Collection under reference number ATCC 74135

The lovastatin is produced in association with the hydroxy acid analog thereof, which is readily convertible to lovastatin e.g. by refluxing in toluene, to accomplish lactonization and thereby to increase the lovastatin yield. The lactonization was not undertaken in these experiments.

Lovastatin produced by A. terreus parent culture in Czapek'dox broth was 166.72 ± 5.92 mg/l, and parent A. oryzae did not produce any lovastatin.

Figure 2 of the accompanying drawings is the $^{1}$H-NMR spectrum of the lovastatin compound purified by HPLC from the hybrid strain. By comparison with the known, standard spectrum of lovastatin, this spectrum confirms the identity of the product.

Figure 3 of the accompanying drawings is the mass spectrum of the same product, and shows that lovastatin was obtained at a purity of 99%.

Concentrations of DNA ranging from 5-20 ng per ml of medium affected neither the regeneration capacity of the A. oryzae protoplasts nor the recovery of cycloheximide resistant and lovastatin-producing isolates from incubations with A. terreus DNA. The data indicate that these concentrations were not a limiting factor in the production of lovastatin. Similarly, DNA from calf-thymus, DNA and homologous A. oryzae DNA at concentrations from 5 ng to 5 ug per ml of medium did not affect the regeneration of A. oryzae protoplasts when compared with untreated controls. However, DNA at 10 and 100 ug per ml of medium reduced regeneration to maximum of 26% and 1% respectively. These DNA treatments did not elicit lovastatin expression.

The regenerating medium yielded numbers of protoplasts comparable to those obtained by Acha et al. J. Gen. Microbiol., 45, 515-523 (1966), using a more complex mineral medium. Conidia and freshly germinated conidia produced better yields of viable protoplasts and of DNA than the mycelia. The formation of cellular aggregates during protoplast regeneration described by Acha et al. (1966) was not observed in this system. The apparent transfer of factors responsible for the expression of cycloheximide resistance and lovastatin expression from A. terreus to A. oryzae indicates interspecies DNA transformation. The co-transformation of cycloheximide resistance and lovastatin production was unexpectedly satisfactory and suggests a physical proximity and possible clustering of the genes involved in the expression of these characteristics.

The morphology of the novel transformant AoAt-NBJ/5 is depicted in Figure 4 and may be characterized as follows:

MORPHOLOGY OF AoAt-NBJ/5

Conidial heads columnar, light brown. Conidiophores smooth, colourless. Vesicles hemispherical, covered up to one-half or two-thirds by phialides arranged in two layers. Conidia globose or ellipsoidal, smooth colonies on Czapek agar growing very rapidly, either floccose or velvety cinnamon brown due to production of conidia. Orange exudate, reverse yellow to brown.

These experiments demonstrate that, in principle, genetically determined antibiotic producing characteristics of one fungal species can be expressed in another.

EXAMPLE 2 - METHOD OF MANUFACTURE

Fungal culture: A transformed culture of Aspergillus oryzae (ATCC#74135) AoAt/NBJ-V was used to produce lovastatin.

FERMENTATION

Seed preparation: The fungal culture (lyophilized vial) was transferred aseptically onto the Potato-dextrose agar slant and allowed to grow at 25°C for 4-5 days. At the end of incubation, the conidial suspension was prepared by adding 10 ml of triton X-100 (0.01%) solution, agitated vigorously and transferred the conidial suspension onto sterile rice (50 g) contained in an Erlenmeyer flask (250 ml capacity). The flask was supplemented with an additional 10 ml sterile water, agitated vigorously and incubated at 28°C for 5-7 days. At the end of incubation period, sterile water (50 ml) was added.

The conidial suspension was passed through sterile muslin cloth and the filtrate containing (1 x $10^8$ conidia/ml) was transferred onto seed fermenter (50L capacity) with 30 L of seed media. The composition of seed media used was as follows:

D-glucose 20g/L
Malt extract 20g/L
Neo-peptone 3g/L
Tap water 1L

pH 6.8 (adjusted with 10%-NaOH). (Media was sterilized at 121°C for 30 min (15 psi). The seed was allowed to grow for 17-20 hours at 28°C (aeration rate 0.3-0.5 vvm) with 80-100% dissolved oxygen (200 rpm).

PRODUCTION OF LOVASTATIN

The seed (30L) was transferred onto the production fermenter vessel (1,000L, working volume) containing production media. The production media composition was as follows:

| | |
|---|---|
| Lactose | 60g/L |
| Ardamine pH | 10g/L |
| Soy protein | 2g/L |
| KCL | 2g/L |
| $KH_2PO_4$ | 0.8g/L |
| $MnSO_4 4H_2O$ | 0.03g/L |
| Betaine | 0.6g/L |
| $P_{2000}$ | 2ml |
| Tap Water | 1L |

pH 6.8 (before sterilization, adjusted with 105 $NaOH/H_2SO_4$) sterilization at 121°C for 1 hour at 15-20 psi.

After inoculation, the culture was allowed to grow for 7-8 days at 28°C with pH control at 6.0-6.2 (with 10% $H_2SO_4$).

The aeration rate was maintained between 0.7-1.0 vvm (% dissolved oxygen 80-100%).

2ND PRODUCTION STAGE

The 7 day old fermented broth (50L) was transferred aseptically into a seed fermenter (2,000L working volume) containing 1,500L of seed media (composition given above). The seed was allowed to grow for 24 hours at 28°C (200 rpm) D.O. 80-100%). The broth (200L) was transferred aseptically to production fermenter (30,000L) capacity with working volume 20,000L) containing 18,000L of production media. The culture was allowed to grow further 7-9 days at 28°C with pH control at 6.0-6.2. After 60 hours of fermentation, pH was not controlled. The fermented broth was supplemented with feed media (rate 10L/hr) for 5 days. The feed media composition was as follows:

| | |
|---|---|
| D-glucose | 285.7g/L |
| Ardamine pH | 71.4g/L |
| Soy protein | 14.3g/L |
| Tap Water | 1 L |

pH 6.8 (before sterilization) - sterilized at 121°C for 30-45 min. At the end of fermentation, the broth was acidified to pH 4.0 with ION-HCL and centrifuged. The fungal cake as dried at 55°C and subjected to extraction.

EXTRACTION AND PURIFICATION

The dried fungal cake (20kg) was homogenized with cold ethyl acetate (10-15 min) and the homogenized material was refluxed at 80-85°C for 4-6 hours, allowed to cool and filtered. The filtrate was dried in vacuo to a black tarry material (2.5kg - 3kg). The black tarry material was mixed with silica gel (2.5kg) and $CH_2Cl_2$ (5 litre). The $CH_2Cl_2$ was evaporated in vacuo and the silica blended crude tarry material was poured onto a silica gel glass column (100 cm length x 22.5 cm diam) developed with EtOAc:Hexane (1:2 v.v). Samples (2,000 jl, 12x) were collected. After 24 hours the eluting solvent mixture was changed to EtOAc:Hexane (1:1,v/v) and run for another 24 hours. Samples containing the product were pooled, dried (280g) and crystallized with methanol.

CRYSTALLIZATION

The yellow dried product (280 g.) was dissolved in 2.8L of methanol and boiled for 40-60 minutes at 60-65°C. The hot methanol mixture was filtered and the filtrate incubated for 4-6 hours at 4°C. The white crystalline needles were separated from mother liquor and rinsed with cold methanol. The crystals were dried in vacuo, weighed (190g) and kept in dessicator at 4°C.

The process of the present invention can result in lovastatin yields that are high enough to cause rupture of the cell walls of the novel Aspergillus oryzae transformants, so that in this way the step of lysing the cells prior to recovering the lovastatin can be eliminated. This substantially simplifies the downstream recovery and purification process. As described above, the lovastatin according to the present process can be recovered by a solvent extraction process, without the need to form esters, salts etc. thereof during the course of the recovery. Solvent extraction is a much simpler and more economical process for recovery and purification than chromatography.

Whilst the invention has been described in detail by references of specific experiments, it will be understood that it is not restricted thereto. Its scope is defined in the appended claims.

**Claims**

1. A process of preparing lovastatin which comprises fermenting a nutrient medium with a transformed fungal microorganism of an Aspergillus strain selected from the group of species A. oryzae, A. niger, A. nidulans, A. fumigatus, and A. flavus, which strain is incapable of expressing lovastatin but which has been transformed to contain foreign DNA coding for the set of enzymes capable of synthesizing lovastatin, and a selectable marker, and recovering the lovastatin from the nutrient medium.

2. The process of claim 1 wherein the transformed microorganism contains foreign DNA derived from a lovastatin-producing Aspergillus terreus species.

3. The process of claim 2 wherein said Aspergillus strain is a strain of the species A. oryzae.

4. The process of any preceding claim, wherein the foreign DNA introduced into the transformant contains cycloheximide-resistance genes as selectable marker.

5. The process of any preceding claim, wherein the transformant is AoAt-NBJ/5 as described and defined herein.

6. The process of any preceding claim, including the additional step of lactonization of the hydroxyacid analog formed in the fermentation process to lovastatin.

7. The process of any preceding claim, wherein recovery of the lovastatin is accomplished by solvent extraction.

8. Novel fungal transformants capable of the set of enzymes capable of synthesizing lovastatin, said transformants being strains of an Aspergillus sp. selected from A. oryzae, A. niger, A. nidulans, A.

fumigatus and A. flavus and which is naturally incapable of lovastatin expression but containing foreign DNA containing genes coding for the set of enzymes capable of synthesizing lovastatin.

**9.** Fungal transformants according to claim 9 wherein the Aspergillus sp. is selected from the group consisting of A. oryzae, A. niger, A. nidulans and A. fumigalis.

**10.** Fungal transformants according to claim 10 wherein the Aspergillus sp. is A. oryzae.

**11.** Fungal transformants according to claim 11 wherein the foreign DNA is derived from a lovastatin-expressing strain of the species Aspergillus terreus.

**12.** Fungal transformants according to claim 12, and comprising the product of protoplast transformation of total DNA from said A. terreus strain into said A. oryzae strain.

**13.** The fungal transformants AoAt-NBJ/5 as described and defined herein.

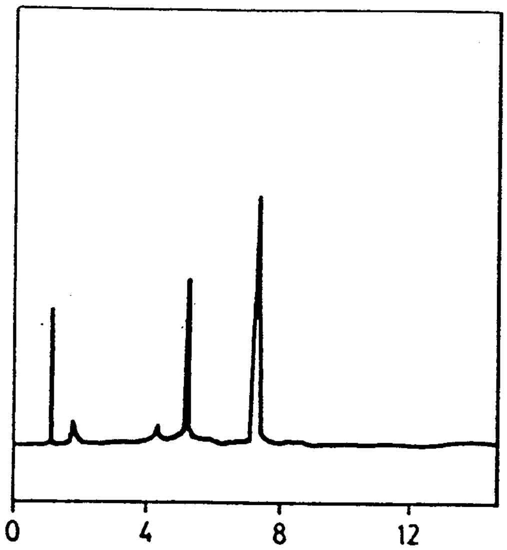


FIG. 1A

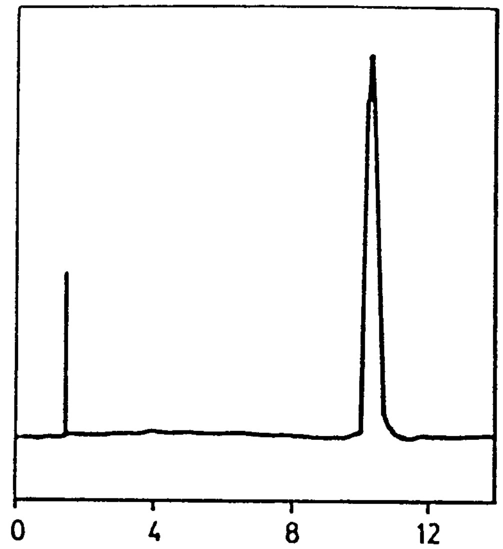


FIG. 1C

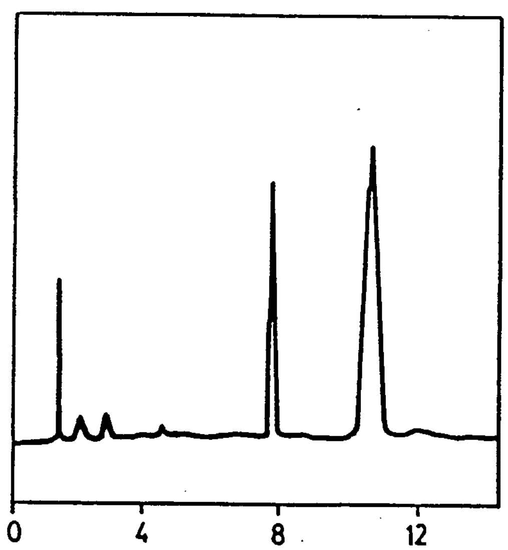


FIG. 1B

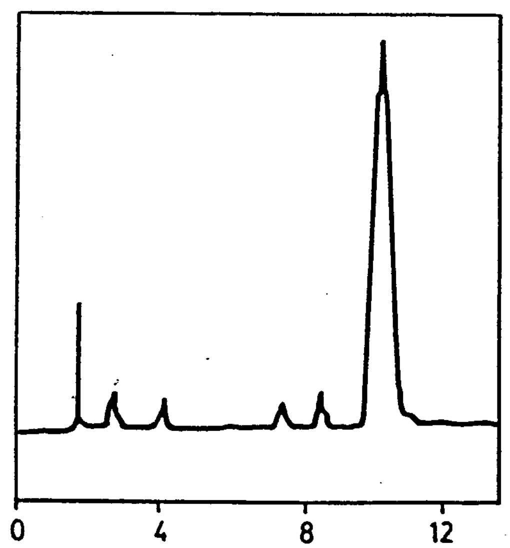


FIG. 1D

A: extract from *A. oryzae* (parent)

B: extract from *A. terreus* (parent)

C: standard lovastatin

D: extract from transformed *A. oryzae*

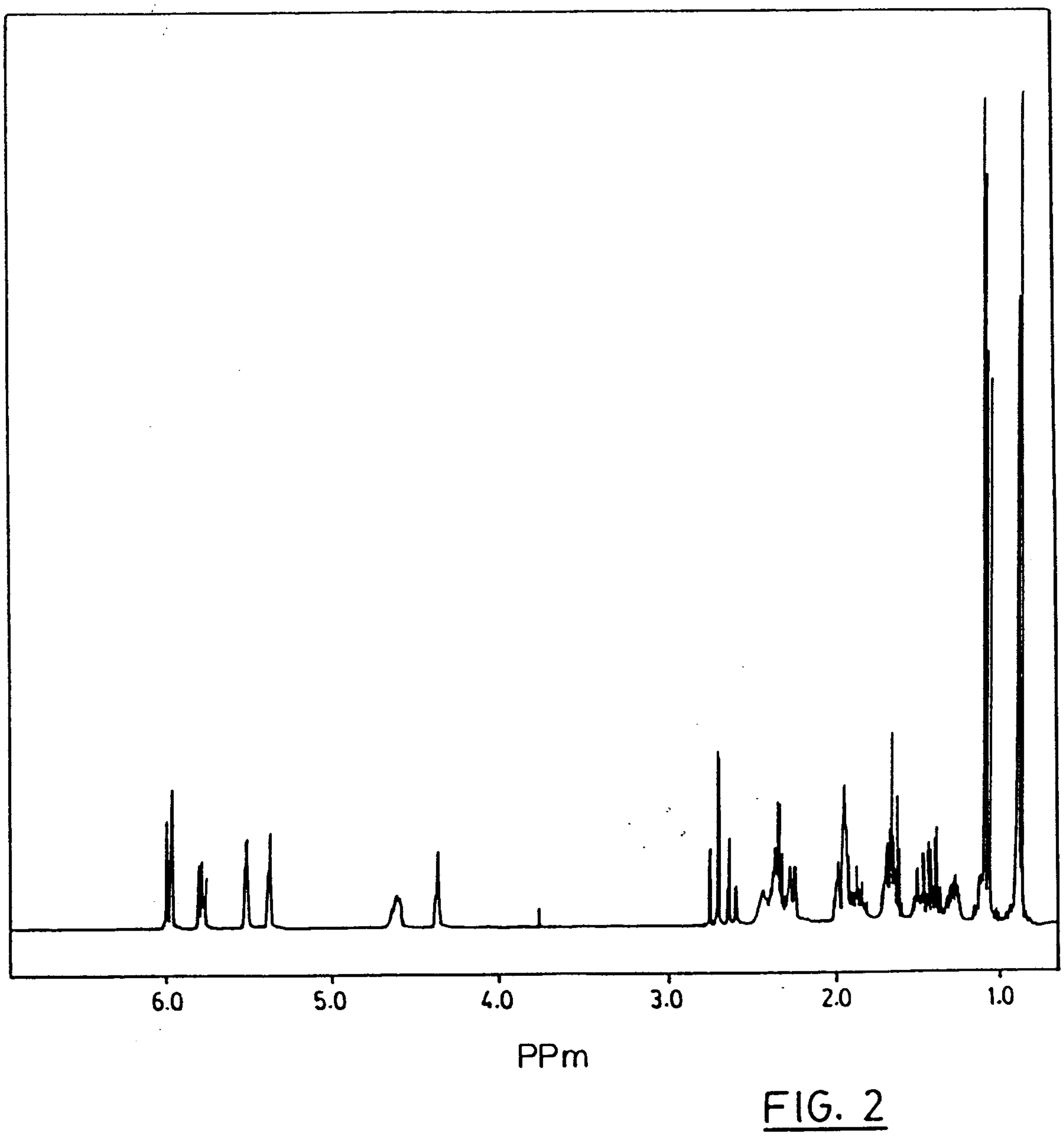


FIG. 2

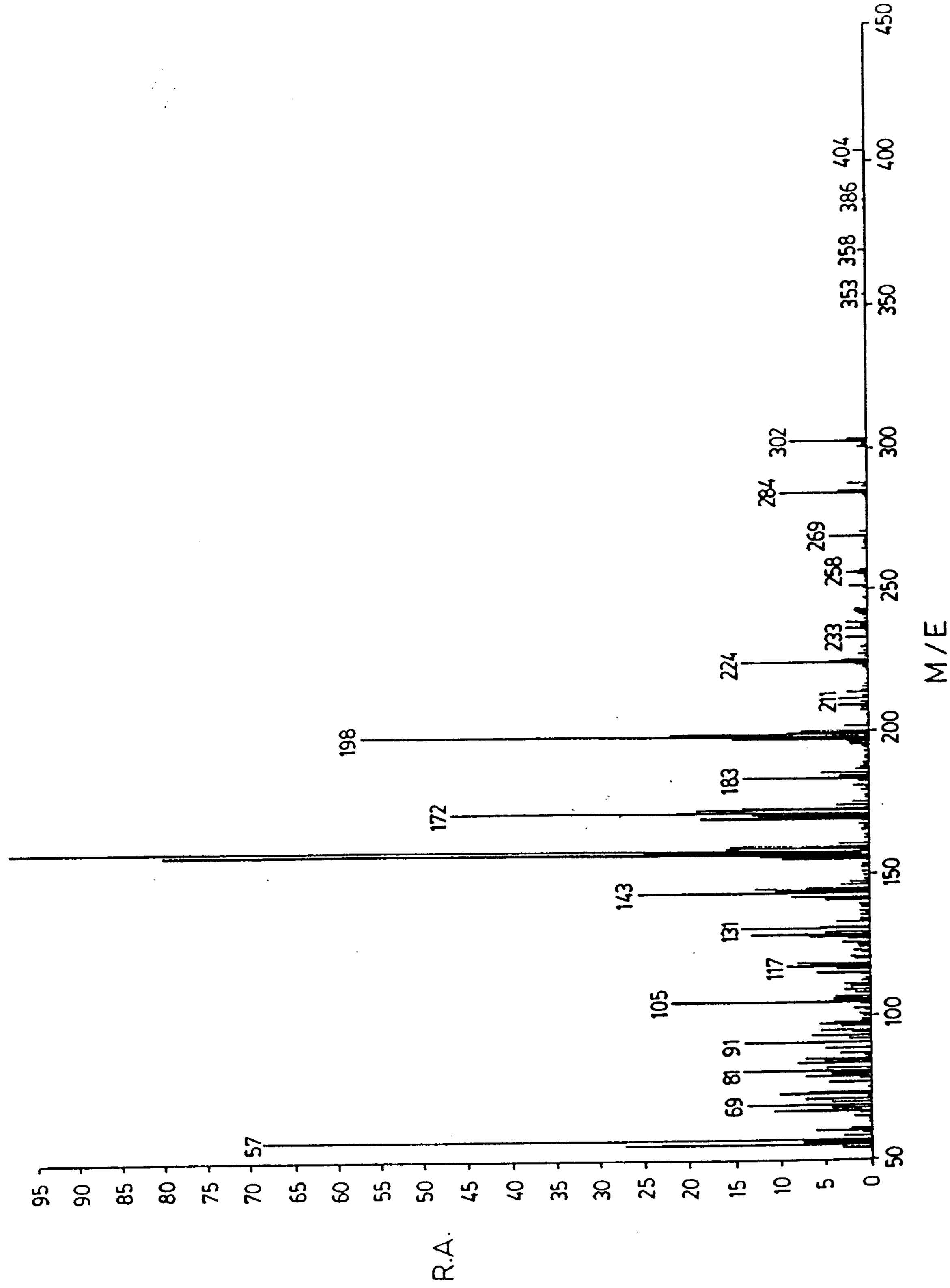
R.A.
M/E
0 5 10 15 20 25 30 35 40 45 50 55 60 65 70 75 80 85 90 95
50 100 150 200 250 300 350 400 450
57 69 81 91 105 117 131 143 172 183 198 211 224 233 258 269 284 302 353 358 386 404

FIG. 3

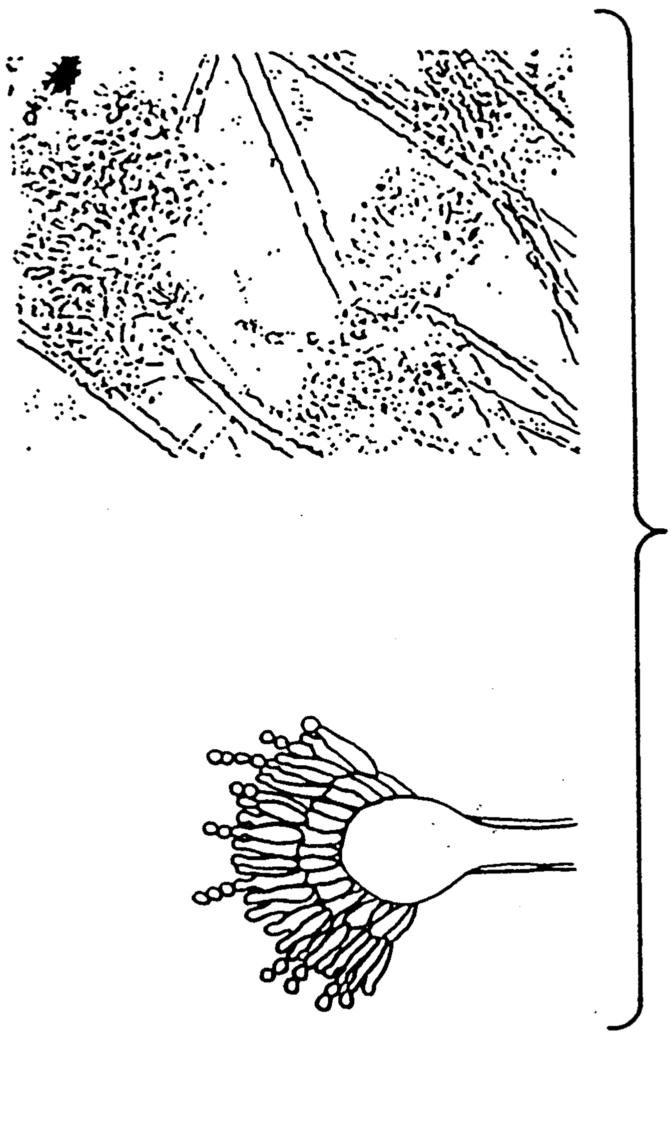

FIG.4

European Patent Office

# EUROPEAN SEARCH REPORT

Application Number

EP 93 10 2016

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 022 478 (MERCK)<br>21 January 1981<br>* claims *<br>--- | 1 | C12N15/52<br>C12P17/06<br>C12N15/80<br>C12P7/42<br>C12N1/15<br>//(C12N1/15,<br>C12R1/66) |
| A | CHEMICAL ABSTRACTS, vol. 115, no. 23,<br>9 December 1991, Columbus, Ohio, US;<br>abstract no. 251890c,<br>VINCI, V.A. ET AL. 'Mutants of a lovostatin-hyperproducing Aspergillus terreus deficient in the production of sulochrin.'<br>page 445 ;<br>* abstract *<br>& J. IND. MICROBIOL.<br>vol. 8, no. 2, 1991,<br>pages 113 - 119<br>--- | 1 | |
| A | EP-A-0 354 624 (GIST-BROCADES)<br>14 February 1990<br>* claims *<br>--- | 1 | |
| A | EP-A-0 463 707 (LUBRIZOL GENETICS)<br>2 January 1992<br>* claims *<br>----- | 1 | TECHNICAL FIELDS SEARCHED (Int. Cl.5)<br>C12P<br>C12N |

The present search report has been drawn up for all claims

| Place of search | Date of completion of the search | Examiner |
|---|---|---|
| THE HAGUE | 25 MAY 1993 | DELANGHE L.L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document